# EUROPEAN PATENT SPECIFICATION

(11) **EP 2 123 296 B1**
(45) Date of publication and mention of the grant of the patent: **22.05.2019**
(21) Application number: 08710659.7
(22) Date of filing: 31.01.2008
(51) Int. Cl.: A61K 39/39, A61K 9/70, A61K 47/32, A61P 37/04

(54) **ADJUVANT FOR TRANSDERMAL OR TRANSMUCOSAL ADMINISTRATION AND PHARMACEUTICAL PREPARATION CONTAINING THE SAME**
HILFSSTOFF FÜR TRANSDERMALE ODER TRANSMUKOSALE VERABREICHUNG UND PHARMAZEUTISCHE ZUBEREITUNG DAMIT
ADJUVANT POUR ADMINISTRATION TRANSDERMIQUE OU TRANSMUCOSALE ET PREPARATION PHARMACEUTIQUE LE CONTENANT

(30) Priority: 31.01.2007 JP 2007022061
(43) Date of publication of application: 25.11.2009
(73) Proprietor: Hisamitsu Pharmaceutical Co., Inc., Tosu-shi, Saga 841-0017 (JP)
(72) Inventor: KUWAHARA, Tetsuji, Tsukuba-shi Ibaraki 305-0856 (JP); TOKUMOTO, Seiji, Tsukuba-shi Ibaraki 305-0856 (JP); MATSUDO, Toshiyuki, Tsukuba-shi Ibaraki 305-0856 (JP)
(74) Representative: Dr. Gassner & Partner mbB
(86) International application number: PCT/JP2008/051499
(87) International publication number: WO 2008/093772

(56) References cited:
- EP-A1- 1 917 976
- WO-A1-02/17961
- WO-A1-2007/015441
- WO-A1-2007/015441
- JP-A- 09 508 614
- JP-A- 10 500 662
- JP-A- 2001 509 491
- JP-A- 2003 509 473
- JP-A- 2004 083 520
- JP-A- 2004 504 120
- JP-A- 2004 510 747
- JP-A- 2004 526 757
- JP-A- 2004 528 900
- US-A- 5 866 157
- US-A- 6 139 866
- US-A1- 2003 203 697
- US-A1- 2004 096 491
- US-A1- 2005 208 116

## Description

### [Technical Field]

The present invention relates to a pharmaceutical preparation containing an adjuvant for transdermal or transmucosal administration for safe and efficient enhancement of immune activity.

### [Background Art]

The skin consists of the stratum corneum that is the outermost layer, an epidermis, a cutis and subcutaneous tissue connecting tissue, and usually, the stratum corneum consisting of the dead cell layer and lipid bilayers shows a strong barrier function for many substances. There is an antigen-presenting cell called Langerhans cells in the epidermis layer, which has an immune function. The mucous membrane is also a border with the outside environment covering an oral cavity, a nasal cavity, respiratory organs, digestive organs, genital organs, and it has the same structure as skin except that there is no stratum corneum that is the outermost layer of the skin. The mucous membrane contacts with various foreign bodies through food intake, breathing, etc. and for example, it is the main passage invading the host body for the pathogenic microorganism. Therefore, the immunological defense mechanism in the mucous membrane is also important as a life barrier.

Langerhans cells capture a protein antigen that invades into skin, disintegrate it inside and express a peptide fragment on an MHC molecule. MHC-peptide complex moves from afferent lymph vessel to the subcortical layer of the regional lymph node, and comes into contact via a T cell and an interdigitating cell. Langerhans cells move in this way, thereby the antigen is conveyed efficiently from skin to a helper T cell (a TH cell) that exists within a lymph node. Langerhans cells are abundant with MHC class II molecules necessary for presenting the antigen to the TH cell.

An adjuvant is a substance that enhances immunogenicity, and when it is administered together with an antigen, a response thereof to the antigen is enhanced. In the vaccination, the adjuvant is useful to reduce a vaccine dose and an administration frequency. Many studies on adjuvants have been made, and as some examples, aluminum salt, immune-stimulating complexes (ISCOM), substances derived from bacteria, etc. are known. However, many of these adjuvants are often directly administered subcutaneously or intramuscularly, and in such cases, the tissue damages, such as contact hypersensitivity, subcutaneous nodule and granuloma are induced. Therefore, in the immunostimulation such as human vaccinations, there is a high demand for adjuvants and preparations that can be administered safely and effectively.

As adjuvants, many vaccine formulations including an attenuated pathogen or the protein subunit antigen have been developed extensively. In most cases, conventional vaccine preparations include adjuvants that enhance immune responses. For example, the adjuvants forming a depot are well known. These adjuvants make the antigen administered be absorbed or settle, which form a depot at an injection position. As typical depot-forming adjuvants, aluminum compounds such as aluminum phosphate and aluminum hydroxide gel, and oil-in-water emulsion etc. are mentioned.

However, the depot-forming adjuvants have a problem in the use, because they bring about local tissue damages such as erythema, contact hypersensitivity and granuloma formation when administered subcutaneously or intramuscularly, while they enhance antigenicity. Furthermore, the problem of the absorbability of the aluminum salt also occurs in the transdermal administration. Such a problem of transdermal absorbability of adjuvants themselves also occurs in an immune-stimulating complex (ISCOM) as an adjuvant, substances derived from bacteria and cytokines. For example, muramyl dipeptide is known to cause a pyrogenic response that is a similar symptom to influenza, or Reiter's syndrome, general arthralgia, and further, in some cases anterior uveitis, arthritis and urethritis at the time of injection, etc.

As above, the conventional adjuvants often caused an intense local tissue damage at the time of subcutaneous administration or intramuscular administration. Therefore, in order to avoid this local tissue damage, the transdermal administration was suggested, but the conventional adjuvants are macromolecules such as an immune-stimulating complex (ISCOM) or the substances derived from bacteria, or an aluminum compound, etc., all of which are compounds that are not suitable for the transdermal administration.

In addition, recently, the external dosage form by iontophoresis or the device equipped with a microneedle as means for increasing penetration has been studied, but at present, if the adjuvant as well as the macromolecular antigen is poorly absorbable, the antigen and the adjuvant cannot be penetrated efficiently.

For example, Patent Publication 1 discloses iontophoresis as a method for delivery of macromolecular antigens into the epidermal cells, but it does not disclose adjuvants.

Patent Publication 2 discloses a skin patch having a microprojection array, a reservoir containing an antigenic agent and an immune response augmenting adjuvant, and a method for use thereof to vaccine animals (for example, humans). However, the adjuvants described in said Patent Publication are only metal salts and macromolecules (peptide, etc.), and it does not describe the adjuvant having the skin permeability.

Patent Publication 3 discloses a low molecular adjuvant in which long-chain fatty alcohols, esters thereof with C1 to C6 alkanoic acids, or certain esters of long-chain fatty acids with alkanols and polyols are administered by infusion, but does not describe the immune response thereof against the antigen by the transdermal administration.

Furthermore, Patent Publication 4 discloses a topical method comprising the step of administering a mixture of an antigen and a lipophilic solvent, and the step of, administering an inducer of the Langerhans cell migration after administration thereof. However, according to the description of said Patent Publication, substances promoting the induction of Langerhans cells are limited to divalent unsaturated carboxylate esters such as dibutyl phthalate represented by the following formula 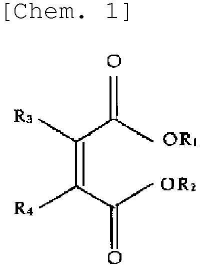 (wherein, R₃ and R₄ may be linked to form a cyclic ring, and R₁ and R₂ are independently alkyl side chains containing from 1 to 16 carbon atoms).

Patent Publication 5 discloses a dry preparation including a cholera toxin or a related ADP-ribosylating toxin as an adjuvant. In such a preparation, the adjuvant of a cholera toxin or a related ADP-ribosylating toxin penetrates the skin, and induces an immune response. On the other hand, there is little information about safety of such an adjuvant, and there are disadvantages that the permeability thereof into the skin is low as it is a high molecule and in addition it is expensive.

Furthermore, Patent Publication 6 discloses a method of immunostimulation by concomitantly using an antigen used for an inoculation, cholera toxin that does not contain an antigen, an adjuvant-containing patch comprising *E. coli* heat-labile enterotoxin vaccine, etc. However, this Patent Publication does not describe the low molecular adjuvant either, and since the adjuvant is a macromolecule and does not penetrate skin easily, the control of the dosage is extremely difficult. In addition, there are disadvantages that it is derived from a toxin, there is also little information about safety, and it is expensive.

As described above, adjuvants used in a conventional injection have problems such as local tissue damages. In addition, transdermal absorption preparation is characterized in that, as compared to an injection agent, it is easy-to-use and excellent in safety, but there are only a few substances effectively showing action of the adjuvants administered transdermally, in particular low molecular compounds, and a method for administration thereof has not been established either. In addition, in clinical practice, the establishment of the adjuvant that can be provided at a low price and safely and the method for administration thereof is strongly desired.
[Patent Publication 1] JP, A, 2002-535100
[Patent Publication 2] JP, A, 2004-538048
[Patent Publication 3] JP, A, 2004-526757
[Patent Publication 4] JP, A, 2002-512186
[Patent Publication 5] JP, A, 2001-517233
[Patent Publication 6] JP, A, 2004-529906

### [Disclosure of Invention]

### [Problems to be Solved by the Invention]

It is therefore an object of the present invention to provide a pharmaceutical preparation comprising a low molecular adjuvant, that can be administered safely without inducing skin irritation etc. by transdermal or transmucosal administration and that is for the purpose of efficiently enhancing the immunogenicity of the antigen. Means for Solving the Problems

While carrying out an intensive investigation in order to solve the above-mentioned problems, the present inventors have found that, certain low molecular compounds show immunoenhancing effects to enhance the immunogenicity of the antigen without causing skin irritation and tissue damage in the transdermal or transmucosal administration, and as a result of further research, created a pharmaceutical preparation comprising these low molecular compounds, and in addition found a method for efficient administration thereof, and the present invention has been accomplished.

That is, the present invention relates to a pharmaceutical preparation that is a patch preparation comprising an immunostimulatory adjuvant for transdermal or transmucosal administration comprising lauryl alcohol, an adhesive of an acrylic copolymer and/or rubber polymer and at least one antigen but not comprising a substance represented by the following formula 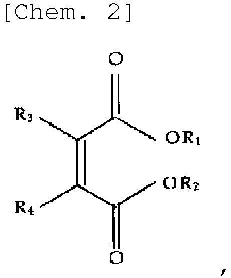 wherein R₃ and R₄ may be linked to form a cyclic ring, and R₁ and R₂ independently represent an alkyl side chain having 1 to 16 carbon atoms.

Further, the present invention relates to a pharmaceutical preparation that is a patch preparation for use as an immunostimulatory adjuvant for transdermal or transmucosal administration, said preparation comprises lauryl alcohol and an adhesive of an acrylic copolymer and/or rubber polymer, but not comprising a substance represented by the following formula 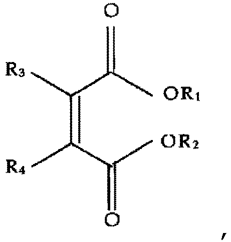 wherein R₃ and R₄ may be linked to form a cyclic ring, and R₁ and R₂ independently represent an alkyl side chain having 1 to 16 carbon atoms.

Moreover, the present invention relates to the patch preparation for use as an immunostimulatory adjuvant for transdermal or transmucosal administration which patch preparation further comprises at least one antigen.

The present invention further relates to the patch preparation for use as an immunostimulatory adjuvant for transdermal or transmucosal administration, which patch preparation is applied to skin or mucous membrane before or after administering antigen, or at the same time with administering antigen.

Moreover, the present invention relates to the patch preparation for use as an immunostimulatory adjuvant for transdermal or transmucosal administration, wherein the patch preparation is at least one selected from the group consisting of a matrix type tape preparation, a laminated type tape preparation and reservoir type patch preparation.

Still further, the present invention relates to the above-described patch preparation for use as an immunostimulatory adjuvant for transdermal or transmucosal administration, which patch preparation is applied to skin or mucous membrane at the same time with administering the antigen, wherein the antigen administration is carried out by puncture administration with a microneedle, and the patch preparation is applied so that the entire microneedle punctured is covered on skin or mucous membrane, thereby the adjuvant can be administered together with the antigen in one step.

The present description discloses not only a method for administering, as a pharmaceutical preparation which further comprises an antigen, the antigen and the adjuvant in the form of an adjuvant pharmaceutical preparation comprising at least one selected from the group consisting of a aliphatic alcohols, free fatty acids and fatty acid derivatives for the transdermal or transmucosal administration, but also a method for transdermal/transmucosal immunostimulation that can enhance the immunogenicity of the antigen safely and efficiently by using said adjuvant pharmaceutical preparation before or after administering an antigen. In addition, the adjuvant generally comprises a compound which molecular weight is lower than conventional adjuvants, thereby enabling transdermal or transmucosal administration.

### Effects of the Invention

(1) The adjuvant can enhance the immunogenicity of the antigen effectively and safely by transdermal or transmucosal administration of the low molecular adjuvant.
(2) The adjuvant comprising lauryl alcohol can enhance the immunogenicity of the antigen efficiently and safely.
(3) The patch preparation of the present invention can enhance immunogenicity of the antigen more easily.
(4) Among the patch preparations for use as an immunostimulatory adjuvant for transdermal or transmucosal administration of the present invention, the one further comprising at least one antigen can enhance the immunogenicity of the antigen more easily and efficiently.
(5) Among the patch preparations for use as an immunostimulatory adjuvant for transdermal or transmucosal administration of the present invention, the one applied to skin or mucous membrane before or after administering the antigen, or at the same time with administering the antigen can enhance the immunogenicity of the antigen even more easily and efficiently.
(6) Among the patch preparations for use as an immunostimulatory adjuvant for transdermal or transmucosal administration of the present invention, the one that is at least one selected from the group consisting of a matrix type tape preparation, a laminated type tape preparation and reservoir type patch preparation enables quicker and long-term delivery of the adjuvant.
(7) Among the patch preparations for use as an immunostimulatory adjuvant for transdermal or transmucosal administration of the present invention, the patch preparation applied to skin or mucous membrane at the same time with administering the antigen, wherein the antigen is administered by puncture administration with a microneedle and the patch preparation is applied so that the entire microneedle punctured is covered on skin or mucous membrane thereby the adjuvant can be administered together with the antigen in one step, enables extremely easy and quick and long-term delivery of the adjuvant in one step at the same time as the puncture administration of the antigen by means of the microneedle, thereby at the same time accomplishing easier, safer and more effectively enhancing action of the immunogenicity of the antigen by the adjuvant.

As above, according to the present invention, a patch preparation comprising a low molecular adjuvant that is extremely excellent in safety does not cause skin irritation and tissue damage in transdermal or transmucosal administration can be provided. Furthermore, the patch preparation of the present invention may be administered in the administration route that is the same as that of the antigen, but may be administered in another administration route that is different from that of the antigen and, in addition, it may be administered at the same time as the antigen administration, but it is not always necessary to administer it at the same time as the antigen administration, so that it becomes possible to exert immunostimulatory activity easily and efficiently by administering it in different administration route from the antigen, or administering it before or after the antigen administration. By providing the patch preparation of the present invention provided with such characteristics, for the sake of the patient with a weak immune response such as senior citizens and immunocompromised people, it becomes possible to reduce a dosage and the frequency of administration of the antigen, and at the same time it becomes possible to bring about the immune response necessary for treatment sufficiently in a shorter term.

Furthermore, the adjuvant for transdermal or transmucosal administration has a low melting point and a low molecular weight, and therefore shows high transdermal or transmucosal absorbability, so that application for preparation for the patch preparation can be realized, and it can be provided at low cost.

### Best Mode for Carrying Out the Invention

The present invention provides a patch preparation comprising an immunostimulatory adjuvant for transdermal or transmucosal administration comprising lauryl alcohol and at least one antigen, but not comprising a substance represented by the following formula

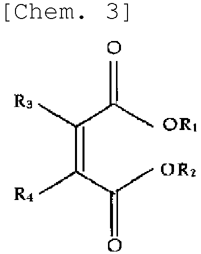

According to the present invention, the term "adjuvant" means a compound that is administered for the purpose of enhancing immunogenicity of an antigen or a vaccine, and in the present specification, it is expressed as "immunostimulatory adjuvant" or merely "adjuvant".

A kind of the ingredient that can be contained in the adjuvant is selected from fatty acid derivatives. The term "fatty acid derivative" means a compound having the fatty acid moiety, and typically fatty acid esters, fatty acid amide, fatty acid halide, etc. are included. Among these fatty acid derivatives, fatty acid esters are preferable, and the fatty acid esters with 10 to 20 fatty acid carbons and degree of fatty acid unsaturation of 0 or 1, and monovalent fatty acid esters, are more preferable respectively.

Such preferable fatty acid esters are, for example, sorbitan monolaurate, propylene glycol monolaurate, sorbitan monooleate, isopropyl myristate, polyethylene glycol, glycerol monooleate, cetyl palmitate and oleyl oleate; in particular, sorbitan monolaurate is most preferable.

Still another ingredient that can be contained in the adjuvant is selected from free fatty acids. Among such free fatty acids, the one with 8 to 20 carbon atoms is preferable. In addition, such fatty acids may be either saturated or unsaturated, linear chain or branched. Among the free fatty acids, preference is given to oleic acid, linoleic acid, γ-linolenic acid, linolenic acid, lauric acid, stearic acid or palmitic acid. Oleic acid and lauric acid are particularly preferable.

The adjuvant may be used alone, or two or more may be used in combination. In particular, in case that there is a synergy effect among the adjuvants, it is preferable that two or more exerting the synergy effect are used in combination. In other cases, the adjuvant may be used alone, and in accordance with the purpose, the adjuvant may be used in combination.

The adjuvant can exert its effect easily by transdermal or transmucosal administration. Therefore, the adjuvant enables the non-invasive body administration in a pharmaceutical preparation in the form of external use by being contained in transdermal or transmucosal administration preparation that is a patch preparation. Such a pharmaceutical preparation is not limited as long as it is in a dosage form that the adjuvant is contained, and is in the form that the adjuvant can be transdermally or transmucosally administered. In addition, in the present specification, a patch preparation includes a matrix type tape preparation, a laminated type tape preparation and a reservoir type patch preparation, and, among these, a matrix type tape preparation and a reservoir type patch preparation are preferably used, a matrix type tape preparation being particularly preferably used.

In the present specification, the matrix type tape preparation refers to the one, among tape preparations, having an adhesive layer in which a pharmacologically active substance is dispersed and contained in a substrate containing essentially a gum-like (glass-like) polymer or a gel provided with adherence, and is provided with a support on the one surface and detachment liner on the other surface of the adhesive layer. In addition, the laminated type tape preparation refers to the one, among tape preparations, having a plurality of adhesive layers in which a pharmacologically active substance is dispersed and contained in a substrate provided with adherence, and affixed with a support on the one surface and detachment liner on the other surface of the adhesive layer. The reservoir type patch preparation refers to the one having a reservoir storing a pharmacologically active substance, and possessing a backing member (a support) that is non-permeable for a drug on the one surface of this reservoir, and possessing a detachment liner or a drug permeable adhesive layer and a detachment liner on the other surface.

In addition, the transdermal or the transmucosal administration preparation of the present invention can be manufactured by a conventional method by using, as the substrate, arbitrary ingredients such as a solubilizer, a solubilizing agent, a pH regulator, a preservative, an absorption accelerator, a stabilizing agent, a filler, a thickener, an adhesive and a wetting agent in combination with the adjuvant. In addition, among pharmaceutical preparations of the present invention, the pharmaceutical preparation of another dosage form can also be manufactured by a conventional method.

For example, among the ingredients of the substrate in the pharmaceutical preparation of the present invention, as a thickener, the one that can stably retain 30% to 80% of moisture and have water retentivity is preferable. As the specific examples thereof, those of plant origin such as guar gum, locust bean gum, carrageenan, alginic acid, sodium alginate, agar, gum arabic, tragacanth gum, karaya gum, pectin and starch, those of microbial origin such as xanthan gum and acacia gum, natural polymers of animal origin such as gelatin and collagen, celluloses such as methyl cellulose, ethyl cellulose, hydroxyethyl cellulose and sodium carboxymethylcellulose, semi-synthetic polymers such as starch like soluble starch, carboxymethyl starch and dialdehyde starch, vinyls such as polyvinyl alcohol, polyvinyl pyrrolidone and polyvinyl methacrylate, acryls such as polyacrylic acid and sodium polyacrylate, as well as water-soluble polymers such as synthetic polymers like polyethylene oxide, and methyl vinyl ether - maleic anhydride copolymer are suitably used. In particular, sodium polyacrylate is preferable. This is because gel strength is high and water retentivity is excellent. Furthermore, sodium polyacrylate having a mean degree of polymerization of 20,000 to 70,000 is preferable. This is because, as the mean degree of polymerization becomes smaller than 20,000, the thickening effect tends to become poor and the gel strength tends to be insufficient, and as the mean degree of polymerization becomes larger than 70,000, the thickening effect tends to be excessively strong, and workability tends to decrease. In addition, by using two or more of the above-described water-soluble polymers together, for example, a polymer complex is formed with the strong ion polymer of sodium polyacrylate, thereby elastic gel with far larger gel strength can be obtained.

As a wetting agent, polyhydric alcohol such as glycerin, propylene glycol and sorbitol may be added, and as a filler, kaolin, zinc oxide, talc, titanium, bentonite, aluminum silicate, titanium oxide, zinc oxide, aluminum metasilicate, calcium sulfate, calcium phosphate, etc. may be added. In addition, as a solubilizing agent or an absorption accelerator, propylene carbonate, crotamiton, 1-menthol, peppermint oil, limonene, diisopropyl adipate, etc. may be added, and as pharmaceutical auxiliaries, methyl salicylate, glycol salicylate, 1-menthol, thymol, peppermint oil, vanilylamide nonylate, red pepper extract, etc. may be added. Furthermore, a stabilizer, an antioxidant, an emulsifier, a surfactant, etc. may be added if necessary.

The surfactant used for a patch preparation of the present invention may be any of non-ionic surfactant and ionic surfactant (cationic, anionic, zwitterionic); from the aspect of the safety, non-ionic surfactant that is usually used for a pharmaceutical substrate is desirable. More particularly, the surfactants are sugar alcohol fatty acid esters such as sucrose fatty acid ester, sorbitan fatty acid ester, glycerin fatty acid ester, polyglycerin fatty acid ester, propylene glycol fatty acid ester, polyoxyethylene sorbitan fatty acid ester, polyoxyethylene glycerin fatty acid ester, polyethylene glycol fatty acid ester, polyoxyethylene castor oil, polyoxyethylene hydrogenated castor oil, etc.

A cross-linking agent, a polymerizing agent, etc. may be added in the patch preparation of the present invention if necessary. This is because a plaster can be made robust as well as water retentive by addition of a cross-linking agent, polymerization agent, etc. This cross-linking agent and the polymerization agent are selected appropriately in accordance with other agents such as thickeners. For example, in case that polyacrylic acid or polyacrylate is employed as a thickener, compounds having at least two epoxy groups in a molecule, oxides, such as inorganic acid salts such as hydrochloride, sulfate, phosphate and carbonate of Ca, Mg, Al, etc., organic acid salts such as citrate, tartrate, gluconate and stearate, zinc oxide and silicic anhydride, polyvalent metal compounds like hydroxides such as aluminum hydroxide and magnesium hydroxide are suitably used. In addition, in case that polyvinyl alcohol is employed as a thickener, adipic acid, thioglycolic acid, an epoxy compound (epichlorohydrin), aldehydes, an N-methylol compound, complexing substances such as compounds of Al, Ti, Zr, Sn, V, Cu, B, Cr, etc. are suitably used. Furthermore, in case that polyvinyl pyrrolidone is employed as a thickener, methyl vinyl ether - maleic anhydride copolymer, a polyacid compound or alkali metal salts thereof (polyacrylic acid and tannic acid and derivatives thereof), etc. are suitably used. In addition, in case that polyethylene oxide is employed as a thickener, peroxides, polysulfone azide, etc. are suitably used.

Furthermore, in case that methyl vinyl ether - maleic anhydride copolymer is employed as a thickener, polyfunctional hydroxy compound, polyamine, iodine, gelatin, polyvinyl pyrrolidone, iron, mercury, lead salt, etc. are suitably used. In case that gelatin is employed as a thickener, aldehydes such as formaldehyde, glutaraldehyde and dialdehyde starch, diepoxides such as glyoxal and butadiene oxide, diketones such as divinyl ketones and diisocyanates are suitably used. In addition, in case that sodium polyacrylate is employed as a thickener, it is preferable that a polyvalent metal salt such as lithium hydroxide, zinc hydroxide, aluminum hydroxide and sodium borate is added as a cross-linking agent. In particular, zinc salt and aluminum salt are preferable. This is because a cross-linking reaction is promoted. The concentration of the polyvalent metal salt added as a cross-linking agent is preferably 0.5 to 1.5 equivalents based on 1 equivalent of the thickener (or water-soluble polymer). This is because, by setting the concentration of polyvalent metal salt to 0.5 equivalent or more, the reaction is promoted and gel strength increases; by setting the concentration of the polyvalent metal salt to 1.5 equivalent or less, the reaction is carried out in a moderate rate and gelation can be equalized, and the workability is enhanced.

As an adhesive used for the patch of the present invention, an acrylic polymer or a rubber polymer is preferable. The acrylic polymer is not particularly limited as long as it is copolymerized with at least one of (meth)acrylic acid derivatives represented by 2-ethylhexyl acrylate, methyl acrylate, butyl acrylate, hydroxyethyl acrylate, 2-ethylhexyl methacrylate, etc.; preferably, the one containing 50% or more of 2-ethylhexyl acrylate is desirable. Specific adhesives are: acrylic acid - octyl acrylate ester copolymer, 2-ethylhexyl acrylate - vinyl pyrrolidone copolymer solution, acrylate ester - vinyl acetate copolymer, 2-ethylhexyl acrylate - 2-ethylhexyl methacrylate - dodecyl methacrylate copolymer, methyl acrylate - 2-ethylhexyl acrylate copolymerized resin emulsion, adhesives such as acrylic polymers included in acrylic resin alkanolamine liquid disclosed in Japanese Pharmaceutical Excipients Directory 2000 (edited by Japan Pharmaceutical Excipients Council) as an adhesive, DURO-TAK acrylic adhesive (manufactured by National Starch and Chemical Company), Eudragit series (Higuchi Inc.), etc. can be used.

The rubber polymers include styrene-isoprene-styrene block copolymer (hereinafter, abbreviated as SIS), isoprene rubber, polyisobutylene (hereinafter, abbreviated as PIB), styrene-butadiene-styrene block copolymer (hereinafter, abbreviated as SBS), styrene-butadiene rubber (hereinafter, abbreviated as SBR), polysiloxane, etc., and among these, SIS, PIB and polysiloxane are preferable, and SIS and PIB are particularly preferable.

Two or more of such hydrophobic polymers may be mixed and used, and the content of these polymers based on the total weight of the composition of these polymers is preferably 5 to 90 percent by weight, and more preferably 10 to 70 percent by weight, taking into consideration the formation of the adhesive layer and sufficient permeability to skin.

A plasticizer may be added to an adhesion matrix (adhesive layer) of the patch of the present invention. The plasticizer that can be used include petroleum oil (for example, paraffin process oil, naphthalene process oil, aromatic process oil, etc.), squalane, squalene, plant oil (for example, olive oil, camellia oil, castor oil, groundnut oil and peanut oil), silicon oil, dibasic acid ester (for example, dibutyl phthalate, dioctyl phthalate, etc.), a liquid rubber (for example, polybutene, liquid isoprene rubber), liquid fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton, etc. are mentioned. Among these, liquid paraffin, liquid polybutene, isopropyl myristate, diethyl sebacate and hexyl laurate are preferable, and in particular, liquid polybutene, isopropyl myristate and liquid paraffin are preferable.

Two kinds or more of these ingredients may be mixed and used, and such a plasticizer can be added in an amount of 10 to 70 percent by weight, preferably 10 to 60 percent by weight, more preferably 10 to 50 percent by weight, based on the total composition of the adhesive layer, taking into consideration the maintenance of sufficient permeability to skin and sufficient cohesive power as the patch.

In case that the adhesive strength is insufficient, it is desirable to add a tackifier resin in the adhesion matrix (the adhesive layer) of the patch of the present invention, and the tackifier resin that can be used includes a rosin derivative (for example, rosin, a glycerin ester of rosin, hydrogenated rosin, a glycerin ester of hydrogenated rosin, pentaerythritol ester of rosin, etc.), an alicyclic saturated hydrocarbon resin (for example, ARKON P100, Arakawa Chemical Industries), an aliphatic 1 series hydrocarbon resin (for example, Quintone B170, Zeon Corporation), a terpene resin (for example, Clearon P-125, Yasuhara Chemical) and a maleic acid resin, etc. In particular, the glycerin ester of hydrogenated rosin, the alicyclic saturated hydrocarbon resin, the aliphatic hydrocarbon resin and the terpene resin are preferable.

Such a tackifier resin can be added in an amount of 5 to 70 percent by weight, preferably 5 to 60 percent by weight, more preferably 10 to 50 percent by weight based on the total composition of the adhesive layer, taking into consideration sufficient adhesive strength as the patch and irritation property to the skin at the time of detachment.

An absorption accelerator may be added to the adhesion matrix (the adhesive layer) of the patch of the present invention, and the absorption accelerator that can be used may be any compound as long as its absorption promotion action on the skin is conventionally recognized; for example, a fatty acid having 6 to 20 carbon atoms, an aliphatic alcohol, a fatty acid ester, amide or ether, an aromatic organic acid, an aromatic alcohol, an aromatic organic acid ester or ether (the above ones may be either saturated or unsaturated, and further may be either cyclic, linear chain, or branched), and further lactate esters, acetate esters, monoterpene compounds, sesquiterpene compounds, azone, azone derivatives, pirotiodecane, glycerin fatty acid esters, propylene glycol fatty acid esters, sorbitan fatty acid esters (Span series), polysorbates (Tween series), polyethylene glycol fatty acid esters, polyoxyethylene hardened castor oil (HCO series), polyoxyethylene alkyl ethers, sucrose fatty acid esters, vegetable oils, etc. are mentioned.

Such absorption accelerators may be used alone or two or more may be mixed and used, and it can be added in preferably 0.01 to 40 percent by weight, more preferably 0.05 to 10 percent by weight, particularly preferably 0.1 to 5 percent by weight, based on the weight of the total composition of the adhesive layer, taking into consideration sufficient permeability to skin as the patch and irritation property to the skin, such as a flare and edema.

In case that an adjuvant is added to the patch preparation, the content of the adjuvant is not particularly limited, but it is preferably the concentration that is sufficient for the adjuvant to exert an immunostimulatory effect as the adjuvant to be absorbed non-invasively into the body. Therefore, the adjuvant is not only used suitably alone, but also preferably the adjuvant is added in 0.1 to 99 percent by weight, more preferably it is added in 5 to 90 percent by weight, in particular, even more preferably it is added in 10 to 80 percent by weight in the patch preparation of the present invention. Most preferably, it is added to the patch preparation of the present invention in 15 to 75 percent by weight.

In addition, the adjuvant can be administered into the body together with an antigen, and therefore the patch preparation of the present invention may further comprise at least one antigen. In this case also, as long as the antigen can be administered transdermally or transmucosally, the transdermal or transmucosal non-invasive patch preparation comprising the adjuvant and the antigen can be prepared. As described above, in the present specification, a patch preparation includes a matrix type tape preparation, a laminated type tape preparation and a reservoir type patch preparation. In addition, such a transdermal or transmucosal preparation of the present invention can also be manufactured by a conventional method by using the arbitrary ingredients such as a solubilizer and a solubilizing agent, a pH regulator, a preservative, an absorption accelerator, a stabilizer, a filler, a thickener, an adhesive as a substrate, which is combined with the antigen and the adjuvant. In addition, the one enhancing permeability of the adjuvant and/or the antigen to the skin or the mucous membrane can be added to the substrate as the above-described absorption accelerator, but the immunogenicity of the antigen can be enhanced sufficiently by the adjuvant even without comprising such an absorption accelerator.

On the other hand, in case that an antigen used together does not have sufficient transdermal or transmucosal activity, only the adjuvant may be administered transdermally or transmucosally, and the antigen used together may be administered non-transdermally or non-transmucosally.

A preferable method for administration of the patch preparation of the present invention is to apply the patch preparation comprising the adjuvant of the present invention before or after the antigen is administered non-transdermally or non-transmucosally, or at the same time as the antigen is administered. In addition, in case that the patch preparation of the present invention is affixed before the antigen is administered non-transdermally or non-transmucosally, the patch preparation of the present invention may be affixed continuously at the time of administering the antigen, and further also after the antigen is administered.

The amount of the antigen and the adjuvant in the combination preparation of the above-described antigen and the adjuvant can be determined appropriately by the combination of the antigen and the adjuvant. In addition, the content of the adjuvant in such a pharmaceutical preparation is not particularly limited, and it exerts a sufficient antigen immune response by transdermal or transmucosal administration. Therefore, the adjuvant is added preferably in 0.1 to 99 percent by weight, more preferably in 5 to 90 percent by weight, particularly preferably in 10 to 80 percent by weight in the combination preparation of the antigen and the adjuvant. The most preferable content of the adjuvant in the preparation is 15 to 75 percent by weight. On the other hand, the amount of the antigen in the combination preparation of the antigen and the adjuvant is preferably 0.01 to 99 percent by weight, more preferably 0.5 to 80 percent by weight, and particularly preferably 5 to 70 percent by weight.

In addition, in the present specification, the antigen means a substance that binds to an antigen receptor on an immune cell and causes an immune response, examples include, without limitation, polynucleotide (DNA vaccine, RNA vaccine) and the vaccine based on a protein, more particularly, antigens in the form of protein, polysaccharide, oligosaccharide, lipoprotein, attenuated or killed viruses such as cytomegalovirus, a hepatitis B virus, a hepatitis C virus, human papilloma virus, a rubella virus and varicella zoster, attenuated or killed bacteria such as pertussis bacteria, tetanus bacillus, diphtheroid, Group A Streptococcus, *Legionella pneumophila, Neisseria meningitidis, Pseudomonas aeruginosa, Streptococcus pneumoniae, Treponema pallidum* and cholera bacillus, and the mixtures thereof. A number of commercially available vaccines comprising an antigenicity action substance may also be used in the present invention. And further, influenza vaccine, Lyme disease vaccine, rabies vaccine, measles vaccine, epidemic parotitis vaccine, varicella vaccine, smallpox vaccine, hepatitis vaccine, pertussis vaccine and diphtheria vaccine, as well as the antigen used in vaccine therapy such as the one for cancer, arteriosclerosis, nervous system disease and Alzheimer's disease are also included. In addition, this antigen may be an allergen substance having the antigenicity (sensitization properties), and various metals and chemical substances are included. For example, in the case of allergy inspection clarifying the antigen of the atopic dermatitis and the treatment, the house dust such as dust and inactivated mites and various kinds of pollen may be used. In addition, the antigen recognized by an inflammatory T cell related to T cell-mediated autoimmune disease or a symptom is also included.

The administration route of these antigens includes but is not particularly limited to; oral, the administration methods by the injection (intramuscular, subcutaneous, intracutaneous), transmucosal and transdermal administration. In the case of transdermal administration, a transdermal administration means in accordance with skin permeability of the antigen and necessary dosage is selected.

By administering the adjuvant with the means same as the one for the above-described antigen or a different transdermal or transmucosal administration means, Langerhans cells of skin or mucous membrane are activated, and transmitted efficiently from skin or mucous membrane to a TH cell present within a lymph node, thereby a high immune response is accomplished. By this, easy evaluation of external pharmaceuticals, cosmetics or antigenicity of an allergen substance, and prevention or treatment by vaccine of, such as infectious disease, cancer and allergy and treatment of the T cell-mediated autoimmune disease, etc. are enabled.

The preferable method for administration of the pharmaceutical preparation of the present invention is the method in which the adjuvant pharmaceutical preparation of the present invention is transdermally or transmucosally administered before or after administering an antigen, or at the same time as administering the antigen, and more preferably, to affix the patch preparation comprising the adjuvant before or after administering an antigen, or at the same time as administering the antigen. In addition, in case that the patch preparation of the present invention is affixed before the antigen is administered non-transdermally or non-transmucosally, the patch preparation of the present invention may be affixed continuously at the time of administering the antigen, and further also after the antigen is administered.

The affixing time of the patch preparation of the present invention is not particularly limited as long as the adjuvant can sufficiently penetrate skin or mucous membrane to exert the effect sufficiently, even in case that affixing is carried out before or after antigen is administered, or even in case that affixing is carried out at the same time as the antigen is administered, but the range between 0.1 and 96 hours is preferable, and the range between 0.5 and 48 hours is more preferable, and the range between 2 and 24 hours is particularly preferable.

The patch preparation comprising the adjuvant or the patch preparation comprising the adjuvant and an antigen concomitantly can be applied to intact skin or mucous membrane, but for the purpose of enhancing transdermal or transmucosal absorbability, it can also be applied to skin or mucous membrane subjected to a physical or chemical treatment, such as a skin abrading treatment or a mucous membrane abrading treatment, a treatment by means of microneedle, laser irradiation, a thermal treatment, an electric field treatment, a magnetic field treatment, a pressure treatment or an alkali treatment. Furthermore, by means of the method using a device such as iontophoresis, electroporation, sonophoresis (a supersonic wave), or in the form of transdermal or transmucosal administration with the device equipped with a microcannula, a microneedle, a needle-free injection, etc., an immune response against the antigen with high safety can be established with even higher efficiency. Among these, it is particularly preferable that transdermal or transmucosal administration is carried out by means of abrading, a microneedle or a needle-free injection. In addition, the above-described administration form is not particularly limited, and the most suitable administering means can be selected in accordance with permeability of the antigen to skin or mucous membrane and a necessary dosage.

An example for coating onto the needle part of the microneedle is described in JP, A, 2004-504120, JP, A, 2004-528900, WO 2005/016440, etc.

One of the preferable methods for immunostimulation using the patch preparation of the present invention is the method in which an antigen is administered using a microneedle by coating the needle part of the microneedle in a part or the entire surface with the antigen, and the patch preparation comprising the adjuvant is administered transdermally or transmucosally before or after the antigen is administered, or at the same time as the antigen is administered, and more preferably, to administer an antigen using a microneedle by coating the needle part of the microneedle in a part or the entire surface with the antigen, and affix the patch preparation comprising the adjuvant before or after administering the antigen, or at the same time as administering the antigen.

Furthermore, among the method in which an antigen is administered using a microneedle by coating the needle part of the microneedle in a part or the entire surface with the antigen, and the patch preparation of the present invention is administered to skin or mucous membrane at the same time as the antigen is administered, particular preference is given to the method in which administration of the antigen is carried out by puncture administration by means of the microneedle, and the whole the punctured microneedle is applied (administered) so that the patch preparation of the present invention covers skin or mucous membrane, thereby the patch preparation comprising the adjuvant is administered in one step together with the antigen. In such a method, each does not have to work differently in order to carry out administration of the antigen and administration of the patch preparation of the present invention, and also, when the microneedle used for the antigen administration is punctured on skin or mucous membrane, the base surface of the side without the needle of the punctured microneedle and the skin or mucous membrane of the part that the microneedle which is adjacent around said base surface is not punctured are covered together by a patch preparation of the present invention to be fixed stably, thereby easy and certain administration is achieved.

### Examples

The present invention is further explained below in more details by means of Examples, but the scope of the present invention is not limited to these Examples.

### Example 1

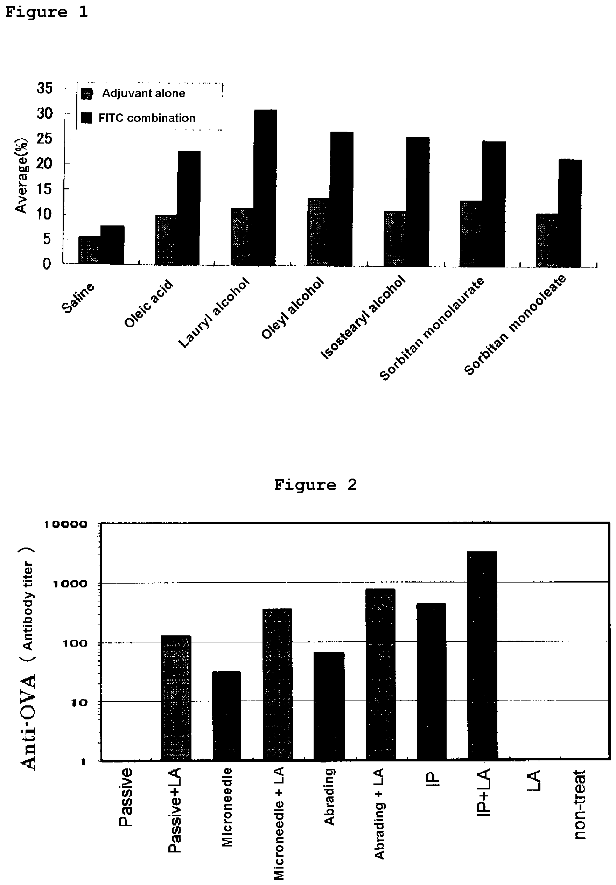

The abdominal hair of a male BALB/c mouse of seven to eight weeks old was shaved and 50 µL of acetone solution (50%) of the candidate adjuvant (oleic acid, lauryl alcohol, oleyl alcohol, isostearyl alcohol, sorbitan monolaurate, sorbitan monooleate) was administered (applied) transdermally (an adjuvant independent group). On the other hand, in the group of combination of an antigen and hapten, 50 µL of 1:1 mixed solution of FITC solution (5 mg/mL in acetone) and each adjuvant solution was administered transdermally in the abdominal region (FITC combination group). Five days later, a lymph node (cervical and inguinal) was extirpated, which was analyzed with flow cytometry for the expression strength of the MHC Class II molecule of the lymph cell (Fig. 1).

As shown in Figure 1, transdermal administration of the low molecular adjuvant of free fatty acid (oleic acid), fatty acid esters (sorbitan monolaurate, sorbitan monooleate) and aliphatic alcohols (lauryl alcohol, oleyl alcohol, isostearyl alcohol) showed remarkable increase of the number of the lymph cells expressing an MHC Class II molecule. That is, it was confirmed that low molecular free fatty acids, fatty acid esters and aliphatic alcohols have a high adjuvant effect in transdermal administration. In particular, the adjuvant effect of lauryl alcohol was the most remarkable.

### Example 2

The abdominal hair of a male BALB/c mouse of seven to eight weeks old was shaved and the degree of the skin irritation of the group to which each 25 µL of the candidate adjuvant (lauryl alcohol, oleyl alcohol, isostearyl alcohol, octyl dodecanol, polyethylene glycol monolaurate, sorbitan monolaurate) (undiluted solution) was administered intracutaneously, and the group to which 50µL of acetone solution (50%) was administered (applied) transdermally, was evaluated with scores (Table 1).

As shown in Table 1, it was confirmed that, in transdermal administration of each low molecular adjuvant of fatty acid esters (polyethylene glycol monolaurate, sorbitan monolaurate) and of aliphatic alcohols (lauryl alcohol, oleyl alcohol, isostearyl alcohol, octyl dodecanol), skin irritation was not detected, and that safety was much higher than intracutaneous administration.

**[Table 1]**

| Skin irritation score (intracutaneous administration versus transdermal administration) | | |
|---|---|---|
| Skin irritation evaluation | Skin irritation score | |
| Adjuvant | Intracutaneous administration | Transdermal administration |
| Lauryl alcohol | +++ | - |
| Oleyl alcohol | +++ | - |
| Isostearyl alcohol | ++ | - |
| Octyl dodecanol | + | - |
| Polyethylene glycol monolaurate | ++ | - |
| Sorbitan monolaurate | ++ | - |

### Example 3

The abdominal hair of a male BALB/c mouse of seven to eight weeks old was shaved, which was divided into untreated group and a group to which the antigen was administered intracutaneously. OVA (an antigen: Ovalbumin, Sigma Company) was prepared by dissolving it in physiological saline to give 10 µg/head, and to the OVA alone group, 25 µL of OVA aqueous solution was administered intracutaneously. To the group in which various kinds of adjuvants (lauryl alcohol, oleyl alcohol, isostearyl alcohol, octyl dodecanol, polyethylene glycol monolaurate and Freund complete adjuvant (FCA)) were used in combination, immediately after OVA solution was administered intracutaneously, 25 µL of each adjuvant solution was administered (applied) transdermally on the abdominal skin surface. Administration was carried out 0, 2 and 4 weeks later, and blood collection was carried out 2, 4 and 5 weeks later, and OVA specific IgG antibody titer after 2, 4 and 5 weeks was measured by ELISA. As the results, data after five weeks are shown in Table 2.

As shown in Table 2, as a result of intracutaneous administration of the antigen or transdermal administration of various kinds of adjuvants to intact skin in combination, and investigating the antigen specific IgG change, a high immune response of the antigen IgG was observed in every adjuvant candidate. The group in which lauryl alcohol and oleyl alcohol were administered in combination showed the highest immune response. From these results, it was confirmed that aliphatic alcohols and fatty acid esters, in particular aliphatic alcohols, not only enhanced transdermal absorbability of the antigen, but also had an immunostimulating action as the adjuvant.

### [Table 2]

**Table 2**

| Adjuvant | Antibody titer (x 10⁴) |
|---|---|
| Lauryl alcohol | 2.1 |
| Oleyl alcohol | 2.1 |
| Isostearyl alcohol | 1.3 |
| Octyl dodecanol | 0.6 |
| Polyethylene glycol monolaurate | 0.9 |
| FCA | 1.3 |
| None | 0.3 |

### Example 4

The abdominal hair of a male BALB/c mouse of seven to eight weeks old was shaved, which was divided into untreated group, microneedle application group, skin abrading pre-treatment group and iontophoresis application group. OVA was prepared to give 100 µg/head, and to the OVA alone group, OVA aqueous solution was applied, and in the group in which lauryl alcohol (LA) was used in combination, the solution in which OVA solution, LA and Tween 20 (emulsifier) was mixed in the ratio of 1:1:0.01 which was emulsified was prepared and used. In the microneedle application group, 1 cm² of a microneedle (needle length: about 200 µm, 400 needles/cm²) was punctured to abdominal skin that had been shaved beforehand, thereafter 50 µL of the above-mentioned emulsion solution was applied immediately. In addition, in skin abrading treatment group, skin was abraded five times using 3M RED Dot™ 2236 instead of a microneedle, thereafter 50 µL of the emulsion solution was applied. In iontophoresis application group, 50 µL of the emulsion solution was applied to shaved abdominal skin, thereafter an iontophoresis preparation (a non-woven fabric preparation: Ag, Ag/AgCl 1 cm²) in which physiological saline was impregnated to the non-woven fabric was affixed in the applied part, and a direct current (0.4 mA/patch) was applied for one hour. Administration was carried out 0, 2 and 4 weeks later, and blood collection was carried out 2, 4 and 5 weeks later, and OVA specific IgG antibody titers were measured by ELISA. With respect to results, data after four weeks are shown in Fig. 2.

As shown in Fig. 2, as a result of inspecting the amount of antigen specific IgG (four weeks later) by transdermal administration of iontophoresis (IP + LA), a microneedle (microneedle + LA), skin abrading pre-treatment (abrading + LA) of the preparation comprising an antigen or an antigen and lauryl alcohol, a high immune response of antigen specific IgG was observed in every transdermal administration form. It was the administration by iontophoresis that showed the highest immune response. In case that lauryl alcohol was used together, significant increase of the antibody titer was observed as compared to the case that an antigen was administered alone.

### Example 5

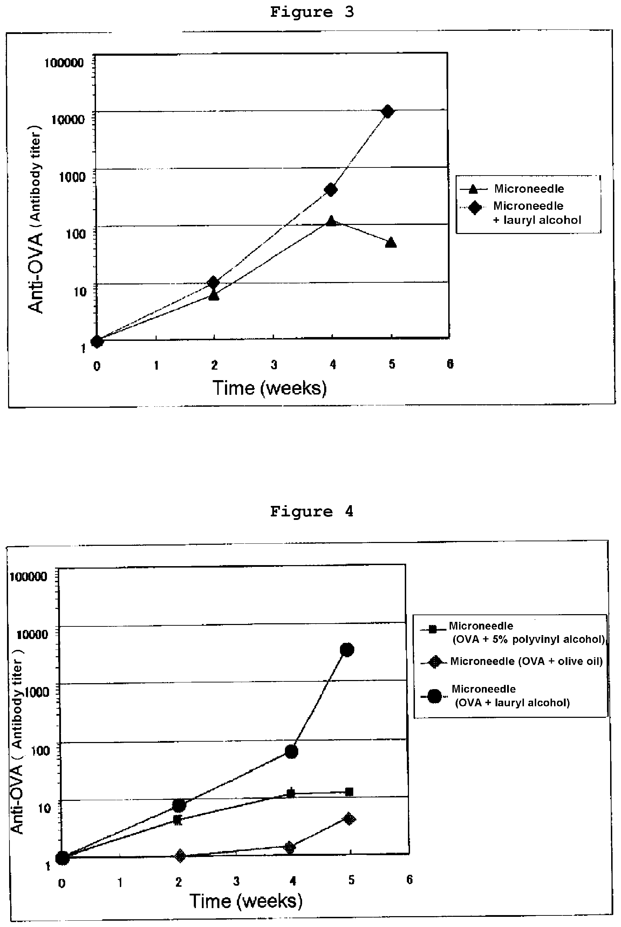

OVA was prepared in the abdomen of the male hairless rat of seven to eight weeks old to give 2 mg/patch, and in the microneedle group, the needle part of the microneedle was coated with an antigen and 5% polyvinyl alcohol liquid, and physiological saline was dropped on the skin side, and punctured for two hours. In addition, in microneedle + lauryl alcohol group, lauryl alcohol was applied to skin beforehand, physiological saline was dropped on the skin side, and the needle of the microneedle coated with the above-described antigen and 5% polyvinyl alcohol liquid was punctured for two hours. Administration was carried out 0, 2 and 4 weeks later, and blood collection was carried out 2, 4 and 5 weeks later, and OVA specific IgG antibody titers were measured by ELISA. Results are shown in Fig. 3.

As shown in Fig. 3, in case that the microneedle coated with an antigen was punctured (a microneedle), antibody titer was not increased, but in case that the microneedle coated with an antigen was punctured after LA was applied to skin before the antigen was administered (microneedle + lauryl alcohol), antibody titer was remarkably increased. Therefore, in the microneedle coated with an antigen, immune enhancing action by using LA together was confirmed.

### Example 6

OVA was prepared in the abdomen of the male hairless rat of seven to eight weeks old to give 2 mg/patch, and in the microneedle (OVA + 5% polyvinyl alcohol) group, the needle part of the microneedle was coated with an antigen and 5% polyvinyl alcohol liquid [mixed in antigen solution:10% polyvinyl alcohol = 1:1], and punctured for two hours. In addition, in microneedle (OVA + olive oil) or (OVA + lauryl alcohol) group, emulsion [antigen solution:olive oil or lauryl alcohol:surfactant (Tween 80) = 1:1:0.01] was coated on the needle of the microneedle and punctured it for two hours. Administration was carried out 0, 2 and 4 weeks later, and blood collection was carried out 2, 4 and 5 weeks later, and OVA specific IgG antibody titers were measured by ELISA. Results are shown in Fig. 4.

As shown in Fig. 4, in case that the microneedle emulsion-coated with an antigen alone or antigen + olive oil was punctured, increase of antibody titer was not observed, but in the microneedle coated using lauryl alcohol, antibody titer was remarkably increased. Therefore, the usefulness of coating emulsion of the antigen + lauryl alcohol on a microneedle was confirmed.

### Example 7

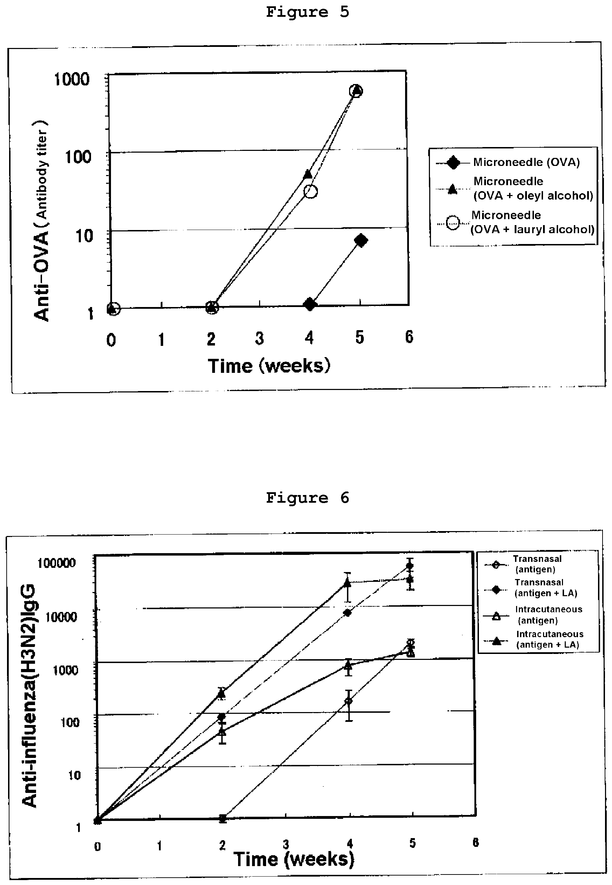

The abdominal hair of a male BALB/c mouse of seven to eight weeks old was shaved, OVA was prepared to give 0.1 mg/patch, and in the microneedle (OVA) group, the needle of the microneedle was coated with an antigen and 5% polyvinyl alcohol liquid [mixed in antigen solution:10% polyvinyl alcohol = 1:1], and punctured for two hours. In addition, in microneedle (OVA + lauryl alcohol or OVA + oleyl alcohol) group, emulsion [antigen solution:lauryl alcohol or oleyl alcohol:surfactant (Tween 80) = 1:1:0.01] was coated on the needle of the microneedle and punctured it for two hours. Administration was carried out 0, 2 and 4 weeks later, and blood collection was carried out 2, 4 and 5 weeks later, and OVA specific IgG antibody titers were measured by ELISA. Results are shown in Fig. 5.

As shown in Fig. 5, in the microneedle coated with an antigen alone, remarkable increase of antibody titer was not observed, but in case that the microneedle emulsion-coated with an antigen + lauryl alcohol or oleyl alcohol was punctured, antibody titer was remarkably increased. Therefore, the usefulness of coating emulsion of the antigen and lauryl alcohol or the antigen and oleyl alcohol on a microneedle was confirmed.

### Example 8

To a male BALB/c mouse of seven to eight weeks old, under inhalation anesthesia (Sevofrane) with matching with breathing, 50 µL of antigen (influenza H3N2 (Biogenesis company): 1 µg/head) was administered to a nose by dripping (transnasal (antigen)). The antigen + LA, [mixed in antigen solution:LA:surfactant (Tween 80) = 9:1:0.05] was administered to a nose by dripping (transnasal (antigen + LA)).

In addition, in the intracutaneous treatment group, the abdomen was shaved under Nembutal anesthesia, 50 µL of antigen (influenza H3N2: 0.07 µg/head) was administered intracutaneously (intracutaneous (antigen)). The antigen + LA, [mixed in antigen solution:LA:surfactant (Tween 80) =1:1:0.01] was administered intracutaneously (intracutaneous (antigen + LA)). Boost was made in the same condition 2 and 4 weeks after the first time administration, and blood was collected from fundus after 2, 4 and 5 weeks and antibody titers were measured. Results are shown in Fig. 6.

In case that emulsion of an antigen and LA was administered transnasally, an increase of the antibody titer was observed in comparison with the transnasal administration of the antigen alone. In the case of the intracutaneous administration, an adjuvant effect by LA was observed as shown previously.

### Example 9

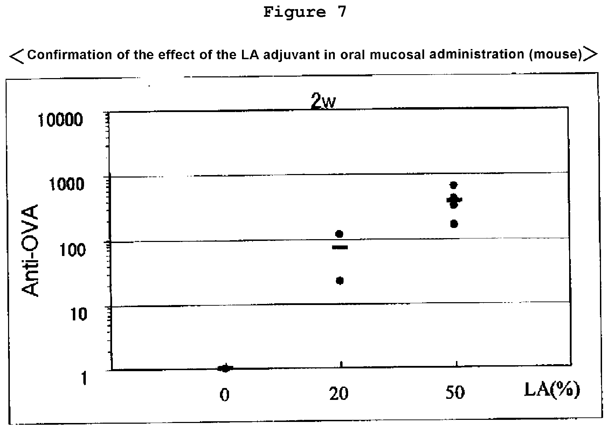

In order to confirm an effect of the LA adjuvant in the oral mucosal administration, each 25 µL was administered twice in the oral cavity of a male BALB/c mouse of seven to eight weeks old (OVA 2500 µg/head. In Fig. 7 showing the results, "0" denotes the treatment group of n=4, 50 µL of dosage [antigen solution], "20" denotes n =2, 50 µL of dosage [mixed in antigen solution:LA:surfactant (Tween 80) = 8:2:0.05], "50" denotes n=4, 50 µL of dosage [mixed in antigen solution:LA:surfactant (Tween 80) = 5:5:0.05], respectively). Boost was made one week after the first time administration, and blood was collected from fundus oculi after two weeks and antibody titers were measured ("-" in Fig. 7 shows the average value of the antibody titers obtained in each treatment group).

In case that two kinds of emulsion, that is, the emulsion of the antibody alone, or the emulsion in which LA concentration was changed, were administered through the oral mucosa, in the group of antigen alone (0), the increase of the antibody titer was not observed, but in the LA combination group ((20) and (50)), the increase of the antibody titer was observed depending on the LA concentration (Fig. 7). In addition, in the present Example, it is considered that a part of the antigen, LA and the surfactant is absorbed through digestive tract mucous membrane, mainly through intestinal tract mucous membrane, and exerts an effect.

### Example 10

The abdomen of a male hairless rat of seven weeks old was shaved, and (1) an acrylic tape preparation added with 40% of lauryl alcohol (LA) (2 cm²) was affixed for six hours or 24 hours, and after detachment, 20 µg/50 µL of OVA (physiological saline) was administered intracutaneously in affixed site (LA tape (6hr) + i.c. or LA tape (24hr) + i.c.), or (2) 20 µg/50 µL of OVA (physiological saline) was administered intracutaneously, thereafter an acrylic tape preparation added with 40% of LA (2 cm²) was affixed for 24 hours (i.c. + LA tape(24hr)). During affixing, a protective tape was used on the upper part of the tape preparation. As controls, (3) the intracutaneous administration group of OVA (i.c.) and (4) the group in which OVA solution and aluminum hydroxide (2mg/mL) were mixed to administer subcutaneously (s.c.(+Alum)) were set. Administration was carried out 0, 2 and 4 weeks later, and blood collection was carried out 2, 4 and 5 weeks later, and OVA specific IgG antibody titers were measured by ELISA.

In addition, the above-described acrylic tape preparation added with 40% of LA (a matrix type tape preparation) was manufactured by mixing 4.0 g of lauryl alcohol, 13.3 g (dry weight: 6.0 g) of Duro-Tak 87-2194 (an acrylic adhesive), spreading the mixture on a detachment liner at thickness of 400 µm, and thereafter drying at 80 °C for 10 minutes, and attaching a support and cutting to 2 cm².

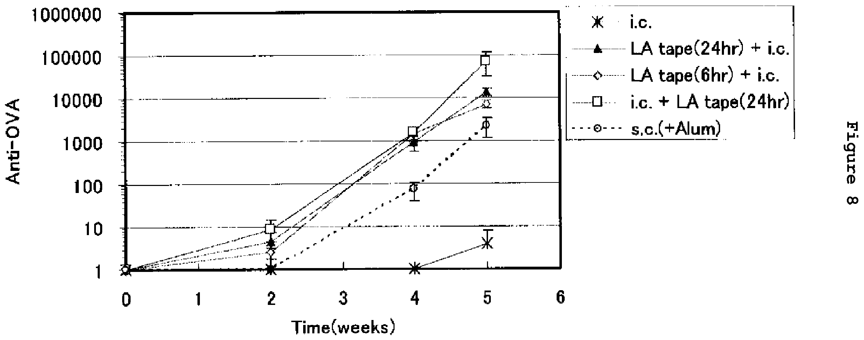

Results are shown in Fig. 8.

As shown in Fig. 8, the antibody titer was not increased only by the intracutaneous administration of OVA, but the antibody titer was increased remarkably by affixing an acrylic tape preparation added with 40% of LA either before or after intracutaneous administration. Therefore, the utility of using a tape preparation added with LA before or after antigen administration was confirmed.

### Example 11

The abdomen of a male hairless rat of seven weeks old was shaved, and (1) an OVA antigen was applied to a microneedle (30 µg/patch) and punctured and administered (only an antigen is applied to MN), (2) a microneedle coated with 25 µL of 30% LA (diluted in olive oil) or 100% LA, and coated with OVA antigen (30 µg/patch) was punctured and administered to an epidermis (30% LA surface application or 100% LA surface application) or (3) OVA antigen was applied (30 µg/patch) to the microneedle, which was punctured and administered, thereafter an acrylic tape preparation added with 40% of LA (2 cm²) was affixed for 24 hours (40% LA tape). As a control, the group to which OVA (30 µg/head) was administered subcutaneously using aluminum hydroxide (2 mg/mL) for the adjuvant (subcutaneous administration (+ Alum)) was set. Administration was carried out 0, 2 and 4 weeks later, and blood collection was carried out 2, 4 and 5 weeks later, and OVA specific IgG antibody titers were measured by ELISA.

In addition, the above-described acrylic tape preparation added with 40% of LA (a matrix type tape preparation) was manufactured by mixing 4.0g of lauryl alcohol, 13.3g (dry weight: 6.0 g) of Duro-Tak 87-2194 (an acrylic adhesive), spreading the mixture on a detachment liner at thickness of 400 µm, and thereafter drying at 80 °C for 10 minutes, and attaching a support and cutting to 2 cm².

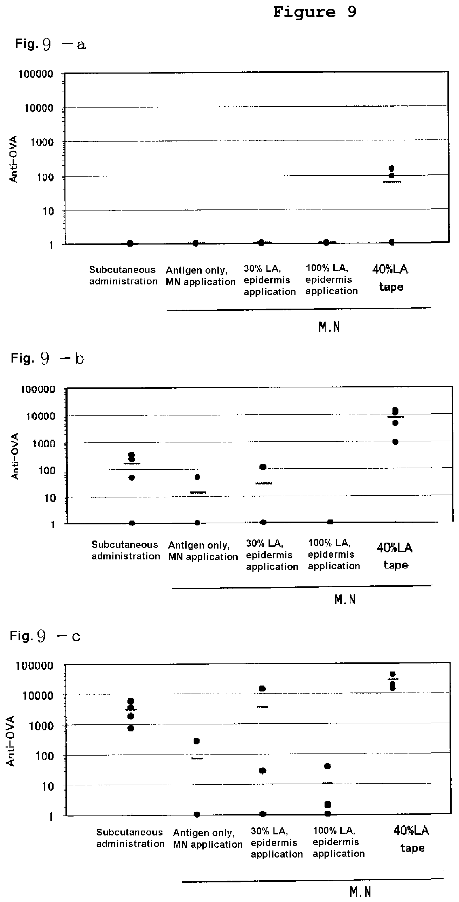

The antibody titers after 2, 4 and 5 weeks are shown in Fig. 9-a, Fig. 9-b and Fig. 9-c, respectively.

It seems that the antigen application of 30 µg/patch to the microneedle in this examination is the administration amount in which an increase of the antibody titer for the animal individual hardly occurs, but as shown in a of Fig. 9, the individual in which the antibody titer increased from two weeks after the first time administration was observed only in the group in which the microneedle administration and the tape preparation added with 40% of LA were used together (40% LA tape). Furthermore, as shown in b of Fig. 9, high antibody titer was observed for all examples in the group in which the microneedle administration and the tape preparation added with 40% of LA were used together (40% LA tape) after the second administration. In addition, in the subcutaneous administration group in which aluminum hydroxide was used together with the adjuvant (subcutaneous administration (+ Alum)), an increase of antibody titer was observed in an individual of some examples, but it was a clearly lower value than LA tape preparation combination group (40% LA tape). After the third administration, as shown in c of Fig. 9, even if various administration groups were compared in which the microneedle administration and the adjuvant were used together, the groups in which an increase of the antibody titer was observed for all examples were the group in which the tape preparation added with 40% of LA was used (40% LA tape) and the subcutaneous administration group (subcutaneous administration (+ Alum)) only, and in addition, the former showed the highest antibody titer. Therefore, even in case that the low dose of the antigen in the degree that usually does not increase the antibody titer is administered, it was shown that the use of the tape preparation including the adjuvant provides sufficiently high antibody titer, and the extremely excellent immunostimulatory effect of the tape preparation of the present invention was confirmed.

### Example 12

The abdomen of a male hairless rat of seven weeks old was shaved, and (1) 20 µg/50 µL of OVA (physiological saline) was administered intracutaneously, and after OVA administration, an acrylic tape preparation added with 40% of LA (2 cm²) was affixed for 2, 6, 12, 24 hours (i.c. 40% acryl 2hr, 6hr, 12hr, 24hr). During affixing of the preparation, protective tape was used on the upper part of the tape preparation. As controls, (2) the intracutaneous administration group of OVA (i.c. (-)), (3) the group in which an acrylic tape preparation without adding the adjuvant was affixed after intracutaneous administration of OVA for 24 hours (i.c. 0% acryl 24hr), (4) the group in which a SIS tape preparation added with 3% of LA or a SIS tape preparation added with 40% of LA was affixed after intracutaneous administration of OVA for 24 hours (i.c. 3% SIS, 40% SIS 24hr), (5) the group in which OVA solution and aluminum hydroxide (2 mg/mL) was mixed to administer subcutaneously (s.c. (+ Alum)) were set. Administration was carried out 0, 2 and 4 weeks later, and blood collection was carried out 2, 4 and 5 weeks later, and OVA specific IgG antibody titers were measured by ELISA.

In addition, the above-described acrylic tape preparation added with 40% of LA (a matrix type tape preparation) was manufactured by mixing 4.0 g of lauryl alcohol, 13.3 g (dry weight: 6.0 g) of Duro-Tak 87-2194 (an acrylic adhesive), spreading the mixture on a detachment liner at thickness of 400 µm, and thereafter drying at 80 °C for 10 minutes, and attaching a support and cutting to 2 cm². In addition, the above-described acrylic tape preparation without adding the adjuvant (a matrix type tape preparation) was produced similarly to the acrylic tape preparation added with 40% of LA except that lauryl alcohol was not added. Further, the above-described SIS tape preparation added with 3% of LA (a matrix type tape preparation) was manufactured by mixing 0.3 g of lauryl alcohol, 2.5 g of SIS (a styrene-isoprene-styrene block copolymer), 3.7 g of alicyclic saturated hydrocarbon resin, 3.5 g of liquid paraffin, 6.7 g of toluene, spreading the mixture on a detachment liner at thickness of 400 µm, and thereafter drying at 80 °C for 10 minutes, and attaching a support and cutting to 2 cm². Similarly, the above-described SIS tape preparation added with 40% of LA (a matrix type tape preparation) was manufactured by mixing 4.0 g of lauryl alcohol, 1.6 g of SIS (a styrene-isoprene-styrene block copolymer), 2.3 g of alicyclic saturated hydrocarbon resin, 2.2 g of liquid paraffin, 6.7 g of toluene, spreading the mixture on a detachment liner at thickness of 400 µm, and thereafter drying at 80 °C for 10 minutes, and attaching a support and cutting to 2 cm².

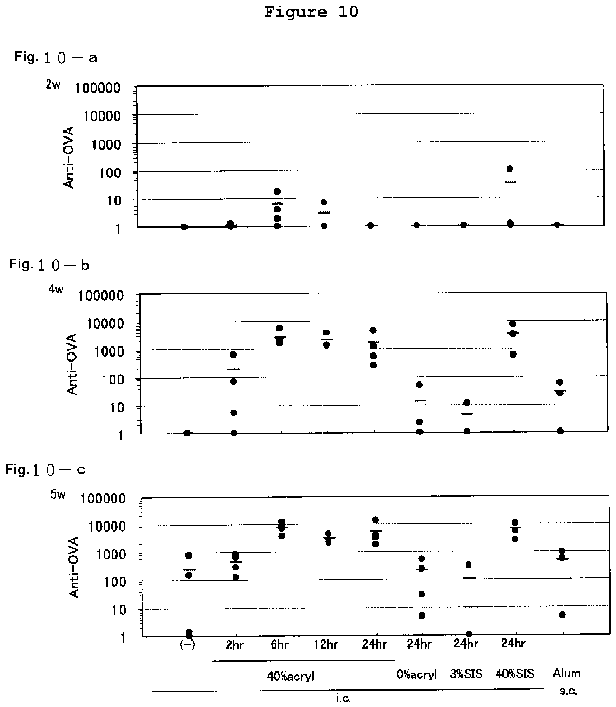

The results after 2, 4 and 5 weeks are shown in Fig. 10-a, Fig. 10-b and Fig. 10-c, respectively.

As is understood by Figs. 10-a to 10-c, when the affixing time of the acrylic tape preparation added with 40% of LA (above-described (1) (i.c. 40%acryl 2hr, 6hr, 12hr, 24hr)) was compared, the antibody titer was increased slightly in all examples by affixing for two hours, and all showed the antibody titer which was extremely high to similar degree by affixing for 6, 12, 24 hours. In addition, in the group in which a SIS tape preparation added with 40% of LA was affixed for 24 hours (above (4) (i.c. 40%SIS 24hr)), it is understood that comparable effect to the group in which the above-described acrylic tape preparation was used is exerted. On the other hand, in the intracutaneous administration group that is the control group (above-described (2) (i.c. (-))) and the group in which the acrylic tape preparation without adding the adjuvant was affixed (above-described (3) i.c. 0%acryl 24hr)), no dramatic increase of the antibody titer was observed even after five weeks. Therefore, it was shown that an excellent immune enhancing effect was obtained by affixing the tape formulation added with 40% of LA for about 6 hours.

From these results, it is considered that the tape preparation of the present invention plays a good immunostimulatory effect by affixing it about 2 hours, and in the case of affixing it about 6 hours or more, an extremely excellent immune enhancing effect is exerted.

### Example 13

The abdomen of a male hairless rat of ten weeks old was shaved, and (1) the group in which an OVA antigen was applied to the microneedle (30 µg/patch) and punctured and administered for two hours (MN2hr), (2) the group in which an OVA antigen was applied to the microneedle (30 µg/patch), punctured and administered, and at the same time an acrylic tape preparation added with 40% of LA (2 cm²) was affixed to cover the entire microneedle, and puncturing and administering by the microneedle was carried out for two hours and affixing of the tape formulation was carried out for six hours (MN2hr+LA tape6hr), (3) the group in which an OVA antigen was applied to the microneedle (30 µg/patch), punctured and administered, and at the same time an acrylic tape preparation added with 40% of LA (5 cm²) was affixed to cover the entire microneedle, and puncturing and administering by the microneedle and affixing of the tape formulation were carried out for six hours (MN6hr+LAtape6hr), (4) the group in which an OVA antigen was applied to the point of the microneedle (30 µg/patch) and at the same time 30% of LA solution was applied on a microneedle substrate, which was punctured and administered for six hours (LAunderMN6hr) were set (in all of each group, n=4). In all groups, the fixation of the microneedle was carried out in Coban and Skinergate. Administration was carried out 0, 2 and 4 weeks later, and blood collection was carried out 5 weeks later, and OVA specific IgG antibody titers were measured by ELISA.

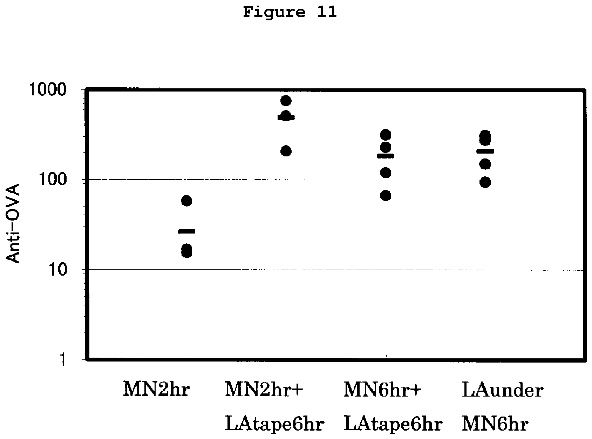

In addition, as the above-described acrylic tape preparation added with 40% of LA (a matrix type tape preparation), the one used in Example 12 was used. Results are shown in Fig. 11.

As shown in Fig. 11, as compared with the case that only an antigen was administered by puncturing by a microneedle (MN2hr), in the case in which the antigen was administered by puncturing by the microneedle and at the same time the tape preparation of the present invention was affixed (MN2hr+LAtape6hr and MN6hr+LAtape6hr), and the case in which the antigen was applied to the point of the microneedle and LA was applied on the substrate of the microneedle, and the antigen and the adjuvant were administered by puncturing at the same time (LAunderMN6hr), more remarkable increase of the antibody titer was observed. Therefore, it was confirmed that the pharmaceutical preparation of the present invention showed an excellent immunostimulatory effect also by administering at the same time with an antigen.

In addition, it was suggested that, in the tape preparation that was applied at the same time with the antigen administration in one step to cover the entire microneedle to be punctured, the antigen administration site and the adjuvant administration site do not always have to be the same as a result of having shown an excellent immunostimulatory effect.

As shown above, it was shown that the adjuvant comprising at least one selected from the group consisting of free fatty acids (oleic acid, etc.), fatty acid derivatives (sorbitan monolaurate, sorbitan monooleate, etc.) and aliphatic alcohols (lauryl alcohol, oleyl alcohol, isostearyl alcohol, etc.) and the pharmaceutical preparation comprising this can remarkably increase the immunogenicity of the antigen easily and safely by administering transdermally or transmucosally at the same time as administering an antigen, or before or after administering an antigen. In addition, it was suggested that, by using the pharmaceutical preparation of the present invention that has an excellent immunostimulatory effect, it becomes possible to reduce the dosage / the administration frequency of the antigen, and besides to reduce a burden to the living body.

### Industrial Applicability

As above, according to the present invention, a patch preparation comprising at least one antigen and an adjuvant for transdermal or transmucosal administration comprising lauryl alcohol for increasing the immune activity safely and effectively is provided. In addition, a method is described to enhance the immunogenicity of the antigen by administering the pharmaceutical preparation of the present invention at the same time as administering an antigen, or before or after administering an antigen. The present invention can be utilized widely for the evaluation of the external pharmaceuticals, cosmetics or allergen substance, vaccine treatment of, such as an infectious disease, cancer, arteriosclerosis, the cranial nerve disease such as the Alzheimer's disease, allergy, etc., and in addition, the use as an antiinflammatory immunomodulator for treating the T cell-mediated diseases is also expected. Therefore, the present invention largely contributes to development of pharmaceutical industry and the related industries thereof.

### Brief Description of Drawings

[Fig. 1] A graph showing an effect of the adjuvant on the expression strength of the MHC-Class II molecule.
[Fig. 2] A graph showing an effect of the adjuvant preparation (containing lauryl alcohol (LA)) on the change of the antigen specific IgG antibody titer (four weeks later) of antigen Ovalbumin in iontophoresis (IP), a microneedle, skin abrading pretreatment (abrading).
[Fig. 3] A graph showing an effect of the adjuvant by using LA together in the microneedle coated with an antigen.
[Fig. 4] A graph showing an effect of the adjuvant by coating emulsion of the antigen + lauryl alcohol on a microneedle.
[Fig. 5] A graph showing an effect of the adjuvant by coating emulsion of the antigen + lauryl alcohol or oleyl alcohol on a microneedle.
[Fig. 6] A graph showing an effect of the adjuvant by transnasal administration.
[Fig. 7] A graph showing an effect of the adjuvant by oral mucosal administration.
[Fig. 8] A graph showing an effect of the pharmaceutical preparation of the present invention (a tape preparation added with LA) affixed intracutaneously before or after administering the antigen.
[Fig. 9] Fig. 9 is a graph showing an effect of the pharmaceutical preparation of the present invention (a tape preparation added with LA) affixed after coating with an antigen and administration by a microneedle (Fig. 9-a shows an effect two weeks later, Fig. 9-b shows an effect four weeks later, and Fig. 9-c shows an effect five weeks later, respectively).
[Fig. 10] Fig. 10 is a graph showing an effect of the pharmaceutical preparation of the present invention (a tape preparation added with LA) affixed after intracutaneous administration of an antigen (Fig. 10-a shows an effect two weeks later, Fig. 10-b shows an effect four weeks later, and Fig. 10-c shows an effect five weeks later, respectively).
[Fig. 11] Fig. 11 is a graph showing an effect of the pharmaceutical preparation of the present invention administered transdermally at the same time as administration of an antigen.

## Claims

1. A patch preparation comprising an immunostimulatory adjuvant for transdermal or transmucosal administration comprising lauryl alcohol, an adhesive of an acrylic copolymer and/or rubber polymer and at least one antigen, but not comprising a substance represented by the following formula 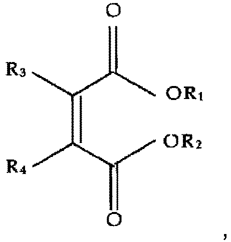 wherein R₃ and R₄ may be linked to form a cyclic ring, and R₁ and R₂ independently represent an alkyl side chain having 1 to 16 carbon atoms.

2. A patch preparation for use as an immunostimulatory adjuvant for transdermal or transmucosal administration, said preparation comprises lauryl alcohol and an adhesive of an acrylic copolymer and/or rubber polymer, but not comprising a substance represented by the following formula 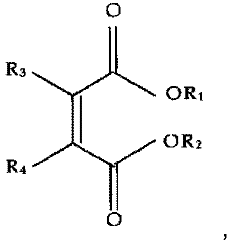 wherein R₃ and R₄ may be linked to form a cyclic ring, and R₁ and R₂ independently represent an alkyl side chain having 1 to 16 carbon atoms.

3. The patch preparation of claim 2 for use according to claim 2, further comprising at least one antigen.

4. The patch preparation of claim 2 for use according to claim 2, which is applied to skin or mucous membrane before or after administering antigen, or at the same time with administering antigen.

5. The patch preparation of any one of claims 2 to 3 for use according to claim 2, wherein the patch preparation is at least one selected from the group consisting of a matrix type tape preparation, a laminated type tape preparation and reservoir type patch preparation.

6. The patch preparation of claim 2 for use according to claim 2, said preparation applied to skin or mucous membrane at the same time with administering the antigen, wherein
antigen administration is carried out by puncture administration with a microneedle, and
the patch preparation is applied so that the entire microneedle punctured is covered on skin or mucous membrane, thereby the adjuvant can be administered together with the antigen in one step.

7. The patch preparation of claim 2 for use according to claim 2, wherein the adjuvant is added in 0.1 to 99 percent per weight.

8. The patch preparation of claim 2 for use according to claim 2, wherein the adjuvant is added in 15 to 75 percent per weight.

## Patentansprüche

1. Pflasterzubereitung, umfassend ein immunstimulatorisches Adjuvans zur transdermalen oder transmukosalen Verabreichung, umfassend Laurylalkohol, einen Klebstoff aus einem Acryl-Copolymer und/oder Gummipolymer und zumindest ein Antigen, jedoch nicht umfassend eine durch die folgende Formel dargestellte Substanz 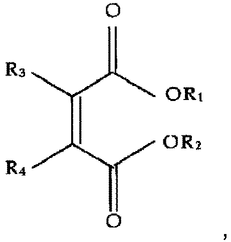 wobei R₃ und R₄ verbunden sein können, um einen zyklischen Ring zu bilden und R₁ und R₂ unabhängig eine 1 bis 16 Kohlenstoffatome aufweisende Alkyl-Seitenkette darstellen.

2. Pflasterzubereitung zur Verwendung als ein immunstimulatorisches Adjuvans zur transdermalen oder transmukosalen Verabreichung, wobei die Zubereitung Laurylalkohol und einen Klebstoff aus einem Acryl-Copolymer und/oder Gummipolymer umfasst, jedoch keine durch die folgende Formel dargestellte Substanz umfasst 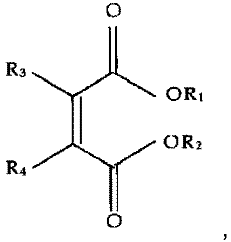 wobei R₃ und R₄ verbunden sein können, um einen zyklischen Ring zu bilden, und R₁ und R₂ unabhängig eine 1 bis 16 Kohlenstoffatome aufweisende Alkyl-Seitenkette darstellen.

3. Pflasterzubereitung nach Anspruch 2 zur Verwendung nach Anspruch 2, weiterhin umfassend zumindest ein Antigen.

4. Pflasterzubereitung nach Anspruch 2 zur Verwendung nach Anspruch 2, welche auf Haut oder Schleimhaut vor oder nach einem Verabreichen von Antigen oder zur gleichen Zeit mit einem Verabreichen von Antigen aufgebracht wird.

5. Pflasterzubereitung nach einem der Ansprüche 2 bis 3 zur Verwendung nach Anspruch 2, wobei die Pflasterzubereitung zumindest eine ist, ausgewählt aus der Gruppe bestehend aus einer Matrixtyp-Bindenzubereitung, einer Bindenzubereitung vom laminierten Typ und einer Reservoirtyp-Pflasterzubereitung.

6. Pflasterzubereitung nach Anspruch 2 zur Verwendung nach Anspruch 2, wobei die Zubereitung zur gleichen Zeit mit Verabreichen des Antigens auf Haut oder Schleimhaut aufgebracht wird, wobei
die Antigenverabreichung durch Punktionsverabreichung mit einer Mikronadel durchgeführt wird und
die Pflasterzubereitung so aufgebracht wird, dass das gesamte Mikronadelpunktierte auf Haut oder Schleimhaut abgedeckt wird, wobei das Adjuvans zusammen mit dem Antigen in einem Schritt verabreicht werden kann.

7. Pflasterzubereitung nach Anspruch 2 zur Verwendung nach Anspruch 2, wobei das Adjuvans in 0,1 bis 99 Gewichtsprozent zugesetzt ist.

8. Pflasterzubereitung nach Anspruch 2 zur Verwendung nach Anspruch 2, wobei das Adjuvans in 15 bis 75 Gewichtsprozent zugesetzt ist.

## Revendications

1. Préparation pour timbre comprenant un adjuvant immunostimulatoire pour l'administration transdermique ou transmucosale comprenant de l'alcool laurylique, un adhésif d'un copolymère acrylique et/ou polymère de caoutchouc et au moins un antigène, mais ne comprenant pas de substance représentée par la formule suivante 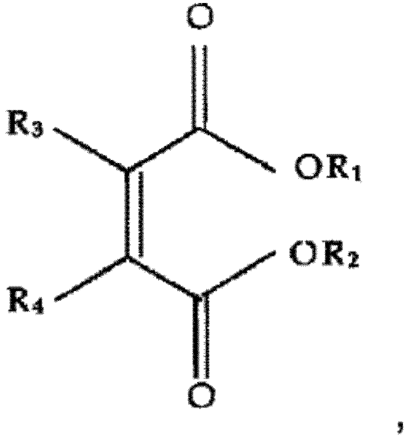 dans laquelle R₃ et R₄ peuvent être liés pour former un anneau cyclique, et R₁ et R₂ représentent indépendamment une chaîne latérale d'alkyle ayant 1 à 16 atomes de carbone.

2. Préparation pour timbre destinée à être utilisée en tant qu'adjuvant immunostimulatoire pour l'administration transdermique ou transmucosale, ladite préparation comprend de l'alcool laurylique et un adhésif d'un copolymère acrylique et/ou polymère de caoutchouc, mais ne comprenant pas de substance représentée par la formule suivante 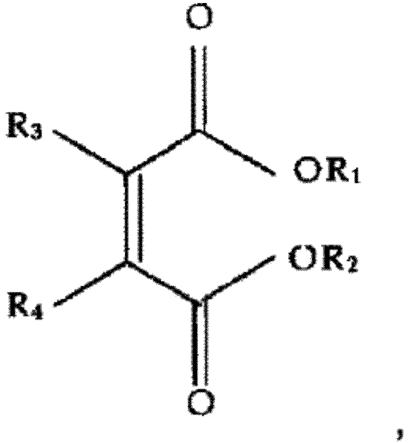 dans laquelle R₃ et R₄ peuvent être liés pour former un anneau cyclique, et R₁ et R₂ représentent indépendamment une chaîne latérale d'alkyle ayant 1 à 16 atomes de carbone.

3. Préparation pour timbre selon la revendication 2 destinée à être utilisée selon la revendication 2, comprenant en outre au moins un antigène.

4. Préparation pour timbre selon la revendication 2 destinée à être utilisée selon la revendication 2, qui est appliquée à la peau ou membrane muqueuse avant ou après l'administration de l'antigène, ou en même temps que l'administration de l'antigène.

5. Préparation pour timbre selon l'une quelconque des revendications 2 à 3 destinée à être utilisée selon la revendication 2, dans laquelle la préparation pour timbre est au moins une sélectionnée parmi le groupe constitué d'une préparation de ruban de type matrice, d'une préparation de ruban de type stratifié et d'une préparation pour timbre de type réservoir.

6. Préparation pour timbre selon la revendication 2 destinée à être utilisée selon la revendication 2, ladite préparation étant appliquée à la peau ou membrane muqueuse en même temps que l'administration de l'antigène, dans laquelle
l'administration de l'antigène est effectuée par administration par ponction avec une microaiguille, et
la préparation pour timbre est appliquée de sorte que la microaiguille entière ponctionnée est recouverte sur la peau ou membrane muqueuse, l'adjuvant peut de ce fait être administré avec l'antigène en une étape.

7. Préparation pour timbre selon la revendication 2 destinée à être utilisée selon la revendication 2, dans laquelle l'adjuvant est ajouté à 0,1 à 99 pourcent en poids.

8. Préparation pour timbre selon la revendication 2 destinée à être utilisée selon la revendication 2, dans laquelle l'adjuvant est ajouté à 15 à 75 pourcent en poids.
